(19) Europäisches Patentamt
European Patent Office
Office européen des brevets

(11) Veröffentlichungsnummer: **0 071 182**
**B1**

(12) # EUROPÄISCHE PATENTSCHRIFT

(45) Veröffentlichungstag der Patentschrift: **10.10.84**

(51) Int. Cl.³: **A 23 L 1/226,** C 07 C 43/205, C 07 C 41/16, C 11 B 9/00

(21) Anmeldenummer: **82106596.8**

(22) Anmeldetag: **21.07.82**

(54) **Verwendung von 1-Ethoxi-4-ethyl-benzol als Riech- und Aromastoff.**

(30) Priorität: **22.07.81 DE 3128987**

(43) Veröffentlichungstag der Anmeldung: **09.02.83 Patentblatt 83/6**

(45) Bekanntmachung des Hinweises auf die Patenterteilung: **10.10.84 Patentblatt 84/41**

(84) Benannte Vertragsstaaten: **AT CH DE FR GB IT LI NL**

(56) *Entgegenhaltungen:*
**GB - A - 1 546 397**

**DIE PHARMAZIE, Heft 4, Seiten 242-245, April 1970, 25. Jahrgang, BERLIN (DE), W.F. FÜRST et al.: "Lipoidmodelle der Arzneimittel-Resorption-Permeation und -Penetration"**
**STEFFEN ARCTANDER: "Perfum and Flavor Chemicals", 1969, Nr. 924, MONTCLAIR N.J. (US)**
**BERICHTE DER DEUTSCHEN CHEMISCHEN GESELLSCHAFT, Band 34, 1901, Seiten 1257-1262, BERLIN (DE), J. MOSCHNER: "Über das Oxy-(4)-hydrinden und einige neue Xylol- und Aethylbenzol-Derivate"**

(73) Patentinhaber: **Dragoco Gerberding & Co. GmbH, Dragocostrasse 1, D-3450 Holzminden (DE)**

(72) Erfinder: **Brunke, Ernst-Joachim, Dr., Holbeinstrasse 6, D-3450 Holzminden (DE)**
Erfinder: **Hoffmann, Paul, Wiesenweg 35, D-3450 Holzminden (DE)**
Erfinder: **Klein, Erich, Dr., Wiesenweg 50, D-3450 Holzminden (DE)**
Erfinder: **Schmidt, Wolfgang, Bebelstrasse 23, D-3450 Holzminden (DE)**
Erfinder: **Warnecke, Hans-Ulrich, Dr., Stadtblick 4, D-3450 Holzminden (DE)**

(74) Vertreter: **Patentanwälte Müller-Boré, Deufel, Schön, Hertel, Lewald, Otto, Postfach 26 02 47 Isartorplatz 6, D-8000 München 26 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die ätherischen Öle von Fenchel (Foeniculum vulgare Mill.), Anis (Pimpinella anisum L.) und Sternanis (Illicum anisatum L.) werden vielfach zur Bereitung von konzentrierten Aroma- oder Parfüm-Mischungen verwendet. In allen den genannten ätherischen Ölen ist als Hauptbestandteil Anethol (2a, b) enthalten (Fenchel: 50—60%, Anis: ca. 90%, Sternanis: 85—90%).

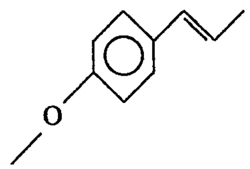
2a (E)

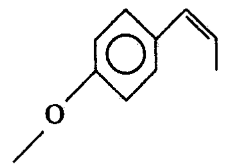
2b (Z)

Bei Einarbeitung der genannten ätherischen Öle in Marktprodukte treten Produktions- und Stabilitätsprobleme auf. Das Anethol erleidet bereits bei Lagerung aber auch bei Einarbeitung in Produkte wie Bonbons, Backwaren usw. eine Autoxidation, die durch Licht, erhöhte Temperatur und Luftzutritt begünstigt wird. Als Autoxidationsprodukt werden die Verbindungen 3—5 gefunden (A. Kraus und F.-J. Hammerschmidt, DRAGOCO-Report 1980, 31). Das Anethol liegt als Gemisch der geometrischen Isomeren 2a und 2b vor, wobei das E-Isomere 2a überwiegendes Hauptprodukt ist. Bei der Lagerung des Öls unter Luftzutritt erhöht sich z. B. der Anteil des Z-Isomeren 2b im Fenchelöl von 0,2% auf 0,5% (90 Tage Raumtemperatur). Bei Bedingungen wie sie bei der Herstellung von Gebäck auftreten, nämlich Erhitzung auf 250° C, erhöht sich der Anteil des Z-Isomeren von 0,2% auf 2,5% (2 Stunden, und bei höherer Temperatur (2 Stunden, 310°C) sogar auf 8,5% (A. Kraus und F.-J. Hammerschmidt, DRAGOCO-Report 1980, 31).

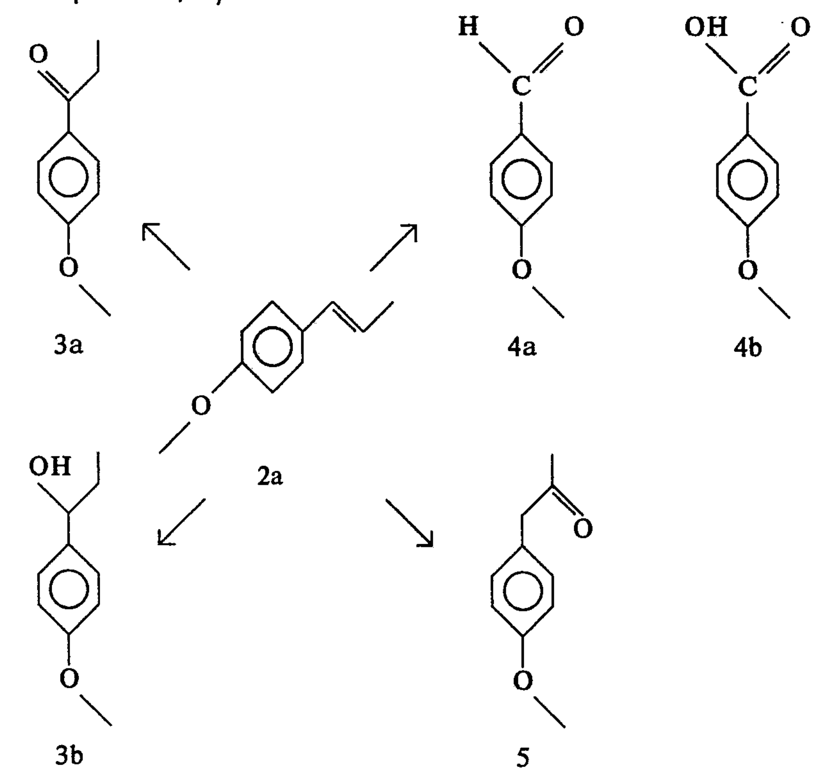
3a 4a 4b

2a

3b 5

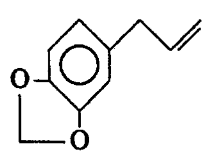
6

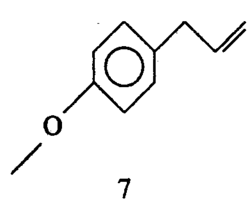
7

Da bekannt ist, daß das Z-Anethol (2b) eine erheblich höhere Toxizität als das E-Isomere 2a besitzt (Y. R. Naves, Parfums, Cosmétiques, Savons 1, 219 [1958]), ist anzustreben, das Z-Isomere 2b zu vermeiden. Dementsprechend ist die Riechstoff- und Aromenindustrie bestrebt, die obengenannten

ätherischen Öle sowie das isolierte oder synthetisierte Anethol mit einem möglichst niedrigen Anteil des Z-Isomeren 2b bereitzustellen. So ist nach den F.D.A. der USA 2b nur in Konzentrationen 0,3% im Aromenkonzentrat erlaubt.

Neuere Untersuchungen haben ergeben, daß das E-Anethol (2a) ebenfalls eine gewisse Toxizität aufweist. So klassifizieren Cramer, Ford und Hall (Food and Cosmetics Toxicology 16, 255—276 [1978]) das Anethol (2a/2b) als Substanz, die nicht als harmlos gelten kann, bzw. sogar signifikante Toxizität vermuten läßt (S. 272). Der Mechanismus der toxischen Wirkung des Safrols (6), einer chemischen dem Anethol verwandten Substanz, wurde kürzlich von Reichert (Angew. Chemie 93, 135 [1981]) beschrieben. Der erste Schritt ist die Oxidation zum 1-Hydroxysafrol, das dem Anethol-Autoxidationsprodukt 3b entspricht. Diese Funktionalisierung ermöglicht die Übertragung körpereigener Substrate durch Transferasen und somit die Bildung der als toxisch und sogar ultimal carcinogen angesehenen Ester (Sulfat, Glucuronid). Eine ähnliche Wirkung wird dem Estragol (7), einem Doppelbindungsisomeren des Anethols, zugeschrieben (Reichert, 1981). Ein ähnlicher Metabolismus ist aufgrund der oben genannten Untersuchungsergebnisse der Autoxidation des Anethols zu befürchten.

Für die Aromen- und Parfüm-Industrie ist es daher wichtig, Ersatzprodukte für das Anethol bereitzustellen, die zwar dessen Geruchs- und Geschmackseffekt erzielen, jedoch nicht die möglicherweise gesundheitsschädigenden Wirkungen des Anethols aufweisen. Da der enzymatische Angriff an der olefinischen Seitenkette erfolgt, sollte ein geeignetes Ersatzprodukt für Anethol eine gesättigte Seitenkette aufweisen. Es wäre daher naheliegend, auf das Dihydro-anethol (8) zurückzugreifen.

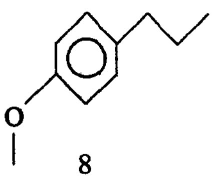

8

Dihydro-anethol ist eine bekannte Substanz, deren Geruch als »anisartig, süß-krautig, holzig, weniger süß und trockener als Anethol« beschrieben wird. Der Geschmack wird als »süß und anisartig, jedoch weniger süß als Anethol« angegeben. Im Gegensatz zum Anethol ist das Dihydro-anethol in Flavour-Mischungen nur in engem Konzentrationsbereich einzusetzen; eine Überdosierung an Dihydro-anethol ist ausgesprochen unangenehm (Arctander, Perfume and Flavour Chemicals, 1969, Nr. 924).

Es ist daher völlig überraschend, daß die Verbindung 1, das 1-Ethoxi-4-ethyl-benzol, einen anisartigen Geruch und einen süß-anisartigen Geschmackseffekt ohne Nebennoten hat, der dem des Anethols sehr nahe kommt. Daher kann das 1-Ethoxi-4-ethyl-benzol (1) als Aromastoff anstelle des Anethols verwendet werden, d. h. als Aromatisierungsmittel, bzw. Bestandteil von Mischungen zur Aromatisierung von Nahrungs- und Genußmittel für den menschlichen Bedarf, wie nichtalkoholische und alkoholische Getränke, Eiscremes, Süßwaren, Gebäck, Süßspeisen, Kaugummi, Zahnpasten, Mundwässer, Gewürzmischungen und andere. Weiterhin kann 1 zur Aromatisierung von Produkten für die Tierernährung verwendet werden.

Auch der Geruchseffekt der Verbindung 1 kommt dem des Anethols sehr nahe. Da die Stabilität der Verbindung 1 in zu parfümierenden technischen Medien, vor allem saure Mischungen, deutlich besser ist als die des Anethols, kann die Verbindung 1 als Riechstoff oder Bestandteil von Riechstoff-Mischungen zur Parfümierung kosmetischer oder technischer Produkte verwendet werden.

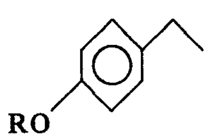

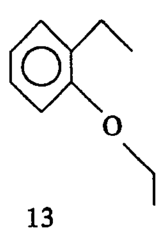

13

9: R = Methyl
10: R = n-Propyl
11: R = i-Propyl
12: R = Allyl

Die durch übliche Veretherung (Natriummethylat, Alkylbromid) dargestellten Homologen 9—12 von p-Ethylphenol besitzen zum Teil ebenfalls Geruchs- und Geschmacksnoten von Anis und Fenchel, jedoch von deutlich geringerer Intensität. Der Geruch der ortho-Isomeren wie 13 weicht vollständig ab. Unter den Verbindungen mit gesättigter Seitenkette besitzt 1 den kräftigsten Anis-Geruch, dessen Intensität nahezu die des Anethols (2a, b) erreicht. Die Verbindung 1 besitzt einen typischen Lakritz-Aspekt, der für die üblichen Anwendungen des Anethols sehr geeignet ist.

Tabelle 1

Geruchsbeschreibung der Verbindungen 1, 2a, b und 8 bis 13

| Substanz | Substituent | Geruch |
|---|---|---|
| 1 | Ethyl | starke, typische Anis-Note, Lakritz |
| 2a, b | | Anis-Note, etwas stärker als 8 und 1 |
| 3 | | anisartig, etwas grün-metallisch, etwas süßer als 1, jedoch weniger Lakritz-Typ |
| 9 | Methyl | Anis, mit metallischer Nebennote |
| 10 | n-Propyl | hell, blumig, ausstrahlend, leicht Anis |
| 11 | i-Propyl | metallisch, blumig, etwas Anis |
| 12 | Allyl | blumig, pilzig |
| 13 | Ethyl(o) | metallisch, krautig, leicht animalisch |

Die Darstellung des 1-Ethoxi-4-ethyl-benzols erfolgt zweckmäßig durch Verätherung von 4-Ethylphenol unter Verwendung von wäßriger Natronlauge und Diethylsulfat. Errera (Gaz. Chim. Italiana 14, 485 [1884]) erhielt die Substanz 1 zusammen mit einer Vielzahl anderer Produkte beim Erhitzen von Phenol mit Ethanol und Zinkchlorid unter Druck. Moschner (Chem. Ber. 34, 1262 [1901]) stellte 1 sowie den analogen Methylether durch übliche Ethylierung des 4-Ethylphenols dar und beschreibt beide Produkte als »etwas anisartig riechende« Flüssigkeiten. Klages und Eppelsheim (Chem. Ber. 36, 3594 [1903]) erhielten 1 durch Reduktion von p-Vinylphenetol mit Natrium und Alkohol. Über den Geschmack war nichts bekannt.

Das nach obiger Methode dargestellte Produkt der Formel 1 besitzt einen hohen Reinheitsgrad und enthält nicht die nach den alten Methoden vorliegenden phenolartigen Verunreinigungen. Demzufolge hat dieses Produkt einen intensiveren und auch qualitativ besseren, anethol-artigen Duft als das nach den älteren Vorschriften dargestellte Material. So ist es auch verständlich, daß bisher weder der olfaktorische noch der sensorische Wert der Verbindung 1 erkannt wurden. Es war somit überraschend, daß das reine 1-Ethoxi-4-ethyl-benzol (1) geeignet ist, anstelle von Anethol (2a, b) in Aromen- oder Riechstoff-Mischungen verwendet zu werden.

## Beispiel 1

### Darstellung von 1-Ethoxi-4-ethyl-benzol (1)

Zu einer Lösung von 1,22 kg (10 mol) 4-Ethyl-phenol in 4,8 l 10% wäßr. NaOH wurden bei ca. 40°C 1,96 kg (13,2 mol) Diethylsulfat zugetropft. Nach 6 Stunden Rühren bei 50°C wurden 2,0 l Wasser zugesetzt. Die Reaktionsmischung wurde 30 min bei Siedetemperatur gerührt, nach Abkühlung mit 3 l Wasser verdünnt und mit Benzin extrahiert. Die vereinigten organischen Phasen wurden mit 2 l 20% wäßr. NaOH ausgerührt (1 Stunde Raumtemp.), mit Wasser gewaschen und eingeengt. Destillation über eine 50-cm-Füllkörperkolonne ergab 1,10 kg (73%) 1 als farblose Flüssigkeit; Kp (2 bar) = 73°C. — NMR ($CCl_4$): $\delta = 1{,}15$, t, J = 7 Hz ($-CH_2-\underline{CH_3}$), 1,25, t, J = 7 Hz ($-O-CH_2-\underline{CH_3}$), 2,47, g ($-\underline{CH_2}-CH_3$), 3,77, g ($-O-\underline{CH_2}-CH_3$), 6,5—7,0, m (aromat. H). — MS: m/e (%) = 150 (34, $M^+$), 135 (31), 122 (5), 107 (100), 91 (7), 77 (13).
$C_{10}H_{14}O$ (150,2)

## Beispiel 2

Parfüm-Öl mit frisch blumiger Note

| | |
|---|---|
| Bergamottöl | 300 g |
| Hydroxycitronellal | 225 g |
| Jasmin-Base*) | 145 g |
| Attractolide*) | 125 g |

| | |
|---|---|
| Citronenöl | 75 g |
| Fichtennadelöl, sibirisch | 50 g |
| Eichenmoos-Extrakt | 15 g |
| Galbanum-Extrakt | 10 g |
| Methylchavicol | 10 g |
| Dimethyltetrahydrobenzaldehyd | 5 g |
| | 960 g |

*) Spezialprodukt DRAGOCO

Die Duftcharakteristik dieses Parfüm-Öles kann mit »Süß-krautig, blumig, in Richtung Maiglöckchen« beschrieben werden. Durch Zugabe von 40 g Anethol (2a, b) wird eine Abrundung unter Betonung der süß-krautigen Aspekte, hingegen durch Zugabe von 40 g 1-Ethoxi-4-ethyl-benzol (1) eine Abrundung unter Betonung »süß-krautiger, sowie aldehydisch-herber« Geruchsnoten bewirkt.

Beispiel 3

Parfümöl mit süß-krautiger Note

| | |
|---|---|
| Orangenöl, brasilianisch (Typ Florida) | 180 g |
| Isobornylacetat | 300 g |
| Cyclogalbanat | 5 g |
| Bergamottöl | 100 g |
| Rosmarinöl, tunesisch | 30 g |
| Beifußöl | 10 g |
| Base Bigaradia 18*) | 15 g |
| Apfelbase*) | 10 g |
| Dimethyl-tetrahydrobenzaldehyd | 5 g |
| Styrolylacetat | 20 g |
| Hexylzimtaldehyd, alpha | 100 g |
| Geraniumöl, Bourbon synth. | 10 g |
| Phenyläthylalkohol | 30 g |
| Citronellol | 100 g |
| Ylanat | 30 g |
| Lignofix*) (Acetylcedren) | 30 g |
| Patchoulyöl, Singapore | 20 g |
| Emanan*) | 5 g |
| Galbanum Resinoid | 10 g |
| Moschus Ambrette | 20 g |
| Attractolide*) | 20 g |
| | 1050 g |

*) Spezialprodukt DRAGOCO

Diesem Parfümöl mit süß-krautiger Note wurden jeweils 50 g Anethol (2a, b), bzw. 50 g 1-Ethoxi-4-ethyl-benzol (1) zugegeben. In den somit erhaltenen neuen Duftkomplexen ergibt die Verbindung 1 ähnliche herb-krautige Effekte wie Anethol, betont aber bei weitem mehr die Kopfnote und führt zu natürlicher Frische im Angeruch.

Beispiel 4

Parfümöl mit herb-krautigen Noten

| | |
|---|---|
| Bergamottöl | 100 g |
| Cedernblätteröl | 150 g |
| Wermutöl | 30 g |
| Rosmarinöl, tunesisch | 45 g |
| Spiköl, spanisch | 20 g |
| Muskatellersalbeiöl | 10 g |
| Pfefferminzöl, brasilianisch | 5 g |
| Phenyläthylalkohol | 100 g |
| Citronellol | 30 g |
| Diphenyläther | 10 g |

| | |
|---|---|
| Geraniumöl Bourbon | 5 g |
| Linalool | 50 g |
| Linalylacetat | 60 g |
| Methyljonon, gamma | 50 g |
| 4-Tert.-butyl-cyclohexylacetat | 50 g |
| Terpineol, rein | 100 g |
| Anisaldehyd, Aubepine | 50 g |
| Lignofix*) (Acetylcedrene) | 100 g |
| Benzylsalicylat | 50 g |
| Amylsalicylat | 70 g |
| Ylang-Ylang-Öl | 30 g |
| Galbanum-Extrakt | 5 g |
| Cumarin | 50 g |
| Attractolide*) | 30 g |
| | 1200 g |

*) Spezialprodukt DRAGOCO

Dieses Parfümöl besitzt einen süßen, herb-krautigen Duftkomplex. Durch die Zugabe von 50 g Anethol (2a, b) bzw. 50 g der Verbindung 1 wird eine Abrundung und Harmonisierung bewirkt. Die Zugabe von Verbindung 1 ergibt zusätzlich eine Betonung der grünen und krautigen Aspekte, d. h. insgesamt eine hellere und spritzigere Kopfnote, die dem Parfüm eine sehr gewünschte moderne Note verleiht.

## Beispiel 5

Geschmacks-Beurteilung und -Schwellenwerte für 2a, b, 1 und 8 (in Zuckerlösung)

| Substanz | Reizschwelle | Erkennungsschwelle | Geschmack |
|---|---|---|---|
| Anethol (2a, b) | 0,10 ppm | 0,12 ppm | typisch Anis |
| Dihydro-anethol (8) | 0,004 ppm | 0,01 ppm | etwas Anis, holzig, muffig, krautig, etwas grün |
| 1-Ethoxi-4-ethyl-benzol (1) | 0,13 ppm | 0,18 ppm | weiche, süße Anis-Note |

## Beispiel 6

Mundpflege-Aromaöl

| | a | b | c |
|---|---|---|---|
| Pfefferminzöl | 620 g | 620 g | 620 g |
| Menthol | 100 g | 100 g | 100 g |
| Krauseminzöl | 100 g | 100 g | 100 g |
| Cineol | 20 g | 20 g | 20 g |
| Nelkenöl | 10 g | 10 g | 10 g |
| Anethol (2a, b) | 150 g | — | — |
| Verbindung 1 | — | 150 g | — |
| | 1000 g | 1000 g | 850 g |

Die drei Mundpflege-Aromaöle wurden in Zahnpasten-Grundmasse eingearbeitet. Die Mischung c besitzt ein frisches, etwas würziges Aroma, das durch Zugabe von Anethol (2a, b) abgerundet wird (Variante a). Bei der Variante b, in der anstelle des Anethols die Verbindung 1 eingesetzt ist, kann eine ähnliche Abrundung wie bei a festgestellt werden, aber auch eine zusätzliche Betonung heller, frischer Aspekte.

Beispiel 7

Gewürzöl, für Honigkuchen

| | a | b |
|---|---|---|
| Anethol (2a, b) | 50 | — |
| Verbindung 1 | — | 50 |
| Zimtöl (Ceylon) | 25 | 25 |
| Nelkenöl, rekt. | 10 | 10 |
| Orangenöl, Florida | 10 | 10 |
| Cardamomöl | 5 | 5 |
| | 100 | 100 |

Die Mischungen a und b besitzen ein kräftiges und typisches Honigkuchen-Aroma. Beide Mischungen sind einander sehr ähnlich, wobei Mischung a etwas süßer und Mischung b etwas heller, frischer wirkt. Honigkuchen, der unter Verwendung von Mischung a oder Mischung b hergestellt wurde, hat in beiden Fällen ein sehr ähnliches, typisches Aroma.

Beispiel 8

Anis-Aroma für Süßwaren

| | a | b |
|---|---|---|
| Anethol (2a, b) | 40 | — |
| Verbindung 1 | — | 40 |
| Orangenöl, rekt. | 10 | 10 |
| Corianderöl | 2,5 | 2,5 |
| Zimtöl (Ceylon) | 2,5 | 2,5 |
| Nelkenöl | 2,5 | 2,5 |
| Angelikawurzelöl | 1,5 | 1,5 |
| Bittermandelöl | 1 | 1 |
| | 60 | 60 |

Die Mischungen a und b lassen sich in Süßwaren wie Bonbons, Hartgummi usw. auf übliche Weise einarbeiten und verleihen dem Produkt ein sehr ähnliches Aroma mit einer ausgeprägten Anis-Note und würzigen Nebennoten. Die Mischung b bewirkt eine leichte Betonung der würzigen Noten.

Beispiel 9

Likör, Typ »Anisette«

| | a | b |
|---|---|---|
| Anethol (2a, b) | 30 | — |
| Verbindung 1 | — | 30 |
| Corianderöl | 4 | 4 |
| Orangenöl (Florida) | 4 | 4 |
| Citronenöl | 4 | 4 |
| Cardamomöl | 2 | 2 |
| Petitgrainöl | 2 | 2 |
| $\beta$-Jonon | 1 | 1 |
| | 47 | 47 |

Die Mischungen a und b wurden jeweils als 4,7%ige Lösung mit Likör-Grundmischung (Ethanol—Wasser 2 : 3, 10% Zucker) angesetzt und ergaben jeweils Liköre mit typischem Anisette-Aroma, wobei die Mischung b durch eine leichte Betonung der Agrumen-Aspekte etwas spritziger war.

Die Verbindung 1 wird zweckmäßig wie folgt angewandt:

a) als Riechstoff (in reiner Form);
b) als Bestandteil von Parfümölen, wobei übliche Konzentrationen 0,1—80%, vorzugsweise 1—20% sind;
c) in Flavour-Mischungen, wobei übliche Konzentrationen 0,1—80%, vorzugsweise 1—40% sind.

**Patentanspruch**

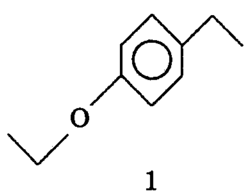

1

Verwendung von 1-Ethoxi-4-ethyl-benzol (1) als Riechstoff und als Aromastoff.

**Claim**

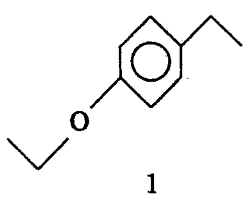

1

Use of 1 Ethoxi-4-ethyl-benzene (1) as odorant and flavoring agent.

**Revendication**

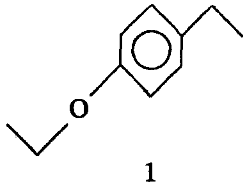

1

Utilisation de Benzol-1-ethoxy-4-ethyl comme matière odorante et matière aromatique.